# EUROPEAN PATENT APPLICATION

(11) **EP 4 130 245 A1**
(43) Date of publication of application: **08.02.2023**
(21) Application number: 21306090.8
(22) Date of filing: 04.08.2021
(51) Int. Cl.: C12N 5/076, A01N 1/02, A61D 19/02, A61K 31/47

(54) **METHOD OF SPERM SORTING USING LIPOSOMES AND/OR AN INHIBITOR OF SLO3 POTASSIUM CHANNEL**

(71) Applicant: Univers 2020, 61300 Saint-Ouen-sur-Iton (FR)
(72) Inventor: CARION, Olivier, 61300 SAINT-OUEN-SUR-ITON (FR); SCHMITT, Eric, 61300 SAINT-OUEN-SUR-ITON (FR)
(74) Representative: Santarelli

(57) **Abstract**

The invention relates to a method of sperm sorting of the spermatozoa contained in the sperm of a mammalian species, said method comprising a step of mixing the sperm sample with liposomes and/or at least one inhibitor of Slo3 potassium channel.

In particular, the invention relates to a method of sperm sorting of the spermatozoa contained in the sperm of a mammalian species, said method comprising the following steps:
- mixing said sperm and a fluorescent dye for labelling sperm DNA with a sample fluid into a fluid mixture;
- providing a flow cytometric sperm sorting device selected from the in-air droplet systems and the flow cell tip close systems;
- using a sperm sorting device on said labelled fluid mixture employing a high energy emitting light source regarding the specific excitation spectrum of the dye, thereby providing each spermatozoon encased in a single droplet of fluid which is hydrodynamically oriented by the use of sheath fluid, identifying the DNA content of said droplet of fluid, assigning an electric charge corresponding to the chromosome status of said droplet and sorting said droplet; and directing said droplet by electrostatic deflection into one of separate collected tubes each containing a catch fluid,
wherein the sample fluid and/or the catch fluid comprises liposomes and/or at least one inhibitor of Slo3 potassium channel.

## Description

The present invention aims the use of a liposome and/or an inhibitor of Slo3 potassium channel, optionally supplemented with at least one additive such as at least one antioxidant, in a method of sperm sorting for mammals including humans, and preferably non-human mammals.

In a particular embodiment, the present invention aims the inclusion of a liposome-containing semen extender and/or an inhibitor of SIo3 potassium channel-containing semen extender, optionally supplemented with at least one additive such as at least one antioxidant, in a method of sperm sorting by fluorescence flow cytometry for mammals including humans, and preferably non-human mammals. Said semen extender is the sample fluid and/or the catch fluid and optionally the sheath fluid used in said method.

The resultant sex-sorted spermatozoa are usually used in assisted reproductive technologies such as artificial insemination or in-vitro fertilization (IVF) to produce offspring of the desired sex.

By "A and/or B", one means here "A, or B, or A and B".

Examples of mammals include, but are not limited to, humans, cattle, pigs, rabbits, horses, goat, sheep, elk, domestic animals such as cats and dogs, and any endangered species.

Examples of non-human mammals include, but are not limited to, cattle, pigs, rabbits, horses, goat, sheep, elk, domestic animals such as cats and dogs, and any endangered species.

In methods of sperm sorting, preservatives such as egg yolk are added to protect sperm during treatment and storage. However, such preservatives may transmit cross species bacterial and/or viral diseases and cannot be standardized in their content and functions. Furthermore, such preservatives make automated diagnoses of sperm characteristics difficult.

There is thus a need to find compounds to replace currently used preservatives that provide best results in terms of protective effects (to protect the sperm sample against mechanical and chemical deterioration after sperm sorting and to protect the sperm membranes such as the plasma and acrosomal membranes) without the drawbacks of absence of standardization and of absence of safety in the current methods using preservatives such as egg yolk.

In particular, sperm sorting may use the technique of flow cytometry to analyze and *"sort"* spermatozoa of domestic animal species. Quantitative flow cytometry employing a droplet in air system or flow-cell type close system (also called in-capillary close system) is the only known method that allows high-purity sorting of sperm according to their sex-related DNA content.

Prior to flow cytometric sorting, sperm are labelled with a fluorescent dye for example a bis-benzimide like the commercial product Hoechst 33342 (trihydrochloride, trihydrate, num. CAS 23491-52-3), which binds to the DNA of each spermatozoon. As the X-chromosome is larger (that is to say, has more DNA) than the Y-chromosome, the "*female*" (X-chromosome bearing) spermatozoa will bind a greater amount of dye than its *"male"* (Y-chromosome bearing) counterpart. As a consequence, when exposed to UV light during flow cytometry, X-spermatozoa fluorescence emission is brighter than Y-spermatozoa. As the spermatozoa pass through the flow cytometer in single-file, each spermatozoon is encased by a single droplet of fluid and an electric charge corresponding to its sex chromosome type (that is to say X-positive charge, Y-negative charge or vice-versa) is assigned. The stream of X- and Y-droplets is then separated by means of electrostatic deflection and collected into separate collection tubes for subsequent processing.

In particular, during the sorting process using the technique of flow cytometry, sperm is in contact with different fluids. It is first in contact with a raw semen extender or sample fluid which is the extender of the raw ejaculate and which further contains the fluorescence dye; followed by a contact with a sheath fluid, which is the medium for hydrodynamic focusing of the core sample stream; and finally it is in contact with a collection medium or catch fluid, which is the fluid used to catch the sorted droplet of sperm.

Besides seminal plasma, each of the current catch fluid as well as the current sample fluid usually contains a buffer, egg yolk, and an energy provider in particular a sugar source, and may include at least one antibiotic and/or antioxidant.

Egg yolk and its components are well known for their protective effects on sperm during treatment and storage. The fact that egg yolk may transmit cross species bacterial and/or viral diseases of avian origin, and that it cannot be standardized in its content and functions, represents a main concern.

Another drawback of egg yolk is related to its irregular molecule composition (sphere-like molecules of different sizes), which makes automated diagnoses of sperm characteristics difficult. It is particularly a problem for using in the catch fluid, where sperm concentration is especially low (typically 0.1 to 2 million spermatozoa per mL in sex sorted batches).

There is a need of an improved sample fluid and/or catch fluid, optionally sheath fluid, in that it provides the same best results; in particular protective effect, as the results obtained with egg yolk, but without the drawbacks of using egg yolk. Therefore said improved fluids should provide the same level of results given by egg yolk for spermatozoa in terms of morphology, motility and viability, but without the drawbacks of absence of standardization and of absence of safety.

There is a need of an improved sample fluid to be used in a sperm sorting process having the following functions:
- Protection of sperm against mechanical and chemical deterioration before sperm sorting ; and
- Protection of the sperm membranes such as the plasma and acrosomal membranes.

There is a need of an improved catch fluid to be used in a sperm sorting process having the following functions:
- A mechanical moderation to break down sperm speed from approximately 50 km/h to about 0 km/h ;
- Neutralization of electric free charges ;
- Protection of sperm against mechanical and chemical deterioration after sperm sorting ; and
- Protection of the sperm membranes such as the plasma and acrosomal membranes.

Advantageously, the present invention discloses a method of sperm sorting that addresses the issues discussed above.

These and other advantages are achieved in accordance with the present invention as described below.

The present invention provides a method of sperm sorting of the spermatozoa contained in the sperm of a mammalian species, said method comprising a step of mixing the sperm of a mammalian species with liposomes and/or at least one inhibitor of SIo3 potassium channel.

Preferably, the sperm of a mammalian species is a sperm of a non-human mammalian species.

In particular, the liposomes and/or at least one inhibitor of SIo3 potassium channel may be used in any liquid media used in methods of sperm sorting.

Examples of methods of sperm sorting include, but are not limited to, any method that could be used to separate X from Y sperm cells such as methods based on:
- DNA content (such as flow cytometry) and/or RNA content (such as flow cytometry),
- Metabolic activity (such as flow cytometry for example through measurement of the mitochondrial potential),
- Expression of cell surface markers (such as flow cytometry for example to reveal cell surface markers that would be differentially expressed in X or Y cells),
- Cellular function (such as motility) in the presence or absence of an exogenous compound (such as static systems that would select X or Y cells based on their differential motility (for example using the swim-up technique) when treated with a receptor ligand regulating motility).

Examples of exogenous compound comprise, but are not limited to, any protein or molecule having an agonistic or inhibitory activity and being able to induce a differential response between X and Y sperm cells.

In a preferred embodiment, the method of sperm sorting is flow cytometry.

The invention also provides a method of sperm sorting of the spermatozoa of a mammalian species, preferably a non-human mammalian species, said method comprising the following steps:
- mixing the sperm of a mammalian species, preferably a non-human mammalian species, and a fluorescent dye for labelling sperm DNA with a sample fluid into a fluid mixture;
- providing a flow cytometric sperm sorting device selected from the in-air droplet systems, and in particular jet-in-air droplet systems, and the flow cell tip (or in-capillary) close systems;
- using said flow cytometric sperm sorting device on said labelled fluid mixture employing a high energy emitting light source according to the specific excitation spectrum of the dye which is hydrodynamically oriented by the use of sheath fluid, assigning an electric charge corresponding to the chromosome status of each sperm cell and sorting said sperm cell by electrostatic deflection into one of two separate collected tubes each containing a catch fluid;
wherein at least one of the sample fluid and catch fluid comprises liposomes and/or at least one inhibitor of SIo3 potassium channel.

In a particular embodiment, said at least one of the sample fluid and the catch fluid is the sample fluid.

In a particular embodiment, said at least one of the sample fluid and the catch fluid is the catch fluid.

The method may also comprise a step of mixing the sperm of a mammalian species, preferably a non-human mammalian species, with liposomes and/or at least one inhibitor of SIo3 potassium channel.

The method may also comprise a step of protecting the sperm of a mammalian species, preferably a non-human mammalian species, prior to labelling such as by using a holding media optionally comprising at least one inhibitor of SIo3 potassium channel and/or liposomes. The at least one inhibitor of SIo3 potassium channel and/or liposomes may be added any time after collection of the sperm sample.

Examples of holding media comprise, but are not limited to, any extender used to dilute crude sperm ejaculate in order to keep spermatozoa in good conditions such as buffer allowing keeping the ejaculate in good condition (pH, osmolarity, antioxidant, in contact with liposomes if any) preferably during 1-12 hours before the beginning of the sorting semen process (such as 1:1 or 1:2 dilution of the ejaculate with respect to buffer).

By "liposomes and/or at least one inhibitor of SIo3 potassium channel" it is herein understood:
- liposomes,
- at least one inhibitor of SIo3 potassium channel, or
- liposomes in combination with at least one inhibitor of SIo3 potassium channel.

By "at least one" it is herein understood one or more, preferably one, two or three.

Inhibitors of SIo3 potassium channel enable to increase the fertility of a mammal in mammal production methods, including artificial insemination methods in which the mammal sperm is in contact with an inhibitor of SIo3 potassium channel. Thus, the present inventors have surprisingly found that the use of SIo3 potassium channel in sperm sorting methods makes it possible to mitigate the drawbacks linked to these methods, thereby obtaining sperm having an increased lifespan and motility to improve the mammal fertility during further insemination methods even if the number of sperm cells in a sexed semen straw are low.

Examples of inhibitors of SIo3 potassium channel according to the present invention comprise, but are not limited to, barium, mibefradil, clofilium and/or quinidine, preferably clofilium and/or quinidine and more preferably clofilium. Barium may be a salt of barium such as barium chloride or barium sulfate. Barium sulfate, which is known as radiocontrast agent, is the inorganic compound with the chemical formula BaSO₄, CAS number 7727-43-7.

Mibefradil may be mibefradil dichloride, the current name of (1S,2S)-2-(2-((3-(1H-benzo[d]imidazol-2-yl)propyl) (methyl)amino)ethyl)-6-fluoro-1-isopropyl-1,2,3,4-tetrahydronaphthalen-2-yl 2-methoxyacetate, known under the trade name Posicor from Roche, CAS number 116644-53-2. It is known as antihypertensive agent and anti-chronic angina pectoris agent.

Clofilium is the current name of 4(-4(Chlorophenyl)butyl-diethyl-heptylammonium (IUPAC name), CAS number 68379-02-2. It is known as an antiarrhythmic agent (class III).

Quinidine is the current name of (9S)-6'-methoxycinchonan-9-ol (IUPAC name), CAS number 56-54-2. It is known as an antiarrhythmic agent (class la).

In particular, clofilium may be clofilium tosylate.

For example, inhibitors of SIo3 potassium channel may be used at a concentration of 0.01 mg/L to 5 mg/L, preferably of 0.02 mg/L to 2 mg/L, more preferably of 0.05 mg/L to 1 mg/L, and even more preferably of 0.1 mg/L to 0.2 mg/L.

In the same manner, the present inventors have surprisingly found that the use of liposomes in sperm sorting methods makes it possible to mitigate the drawbacks linked to these methods, thereby obtaining sperm having an increased lifespan and motility to improve the mammal fertility during further insemination methods even if the number of sperm cells in a sexed semen straw are low.

Liposomes are artificial vesicles (or capsules) formed by concentric lipid bilayers enclosing an aqueous volume. They are obtained from amphiphilic lipids, and especially phospholipid having a polar (hydrophilic) "*head*" generally formed from a glycerol-3 phosphate residue esterified with a polar molecule, and hydrophobic *"tails"* (which are constituted by chains of two fatty acids).

Phosphatidylcholine (or lecithin or 1-oleyl-stearyl-phosphatidylcholine) is a phospholipid derived from egg yolk in a reproducible method.

The method for producing liposomes according to the invention is generally based on extrusion by the means of a mini-extruder such as the one of the company Avanti Polar Lipids. But any other method known to the one skilled in the art can be used. The thus formed vesicles generally have a spheroidal structure, typically of diameter size below 200 nm. The extrusion process is then performed on liposome solution usually through 0.2 µm membrane. The reduction of the liposomes size can be monitored through a dynamic light scattering machine. If needed, the extrusion step is repeated several times to ensure good homogeneity of liposomes in terms of size, as well as to reduce liposome size so that more than 95 % of the liposomes have a diameter of less than 0.2 µm.

This size is well below the size of the spermatozoa to be sorted according to the method of the invention as known to the skilled person.

For example, liposomes may be used at a concentration of 10 g/L to 60 g/L, preferably at a concentration of 30 g/L to 40 g/L.

The liposomes may be formed by phosphatidylcholine in a proportion of 20 % to 100 % by weight, preferably in a proportion of 60 % to 80 % by weight.

In other terms the invention also deals with the use of liposomes and/or of at least one inhibitor of SIo3 potassium channel in semen extender, more specifically in sample fluid and/or catch fluid, for sperm sorting fluid prepared for fluorescent flow cytometry.

According to the invention, the sample fluid and/or catch fluid (i.e. at least one of the sample fluid and the catch fluid) is a pre-determined solution of dilution of semen medium, comprising liposomes and/or at least one inhibitor of SIo3 potassium channel, and usually further comprising a buffer, an energy source (such as fructose, lactate and oxaloacetate as sources of energy for sperm cell) and optionally at least one antibiotic substance, as well as optionally preservative substances including antioxidants for example, to protect sperm membranes of the semen solution (such as egg yolk and catalase). The antibiotic substance is preferably selected, but not limited, from the group consisting of penicillin, gentamycin, streptomycin, and lincomycin. Any other antibiotic substance determined by the European Union regulations in view of bacterial resistance or other reasons can be chosen.

The buffer may also comprise at least one inhibitor of SIo3 potassium channel.

According to a preferred embodiment of the method according to the invention, the sample fluid and/or the catch fluid further comprises at least one antioxidant preferably selected, but not limited, from the group consisting of glutathione, taurine, hypotaurine, pyruvate and cysteine, more preferably glutathione. Said antioxidant is preferably present at a concentration ranged from 1 µM/L to 10 µM/L.

According to preferred embodiments of the method according to the invention,
- The liposomes of the sample fluid and/or the catch fluid are contained in a buffered solution (in other words this means that the sample fluid and/or the catch fluid is(are) buffered solution(s)); and/or
- The liposomes of the sample fluid and/or the catch fluid are present at a concentration of 10 g/L to 60 g/L, preferably at a concentration of 30 g/L to 40 g/L, of said fluid; and/or
- The liposomes of the sample fluid and/or the catch fluid were formed by phosphatidylcholine in a proportion of 20 % to 100 % by weight, preferably in a proportion of 60 % to 80 % by weight, of said fluid; and/or
- More than 95 % of the liposomes of the sample fluid and/or the catch fluid have a diameter of less than 0.2 µm; and/or
- The at least one inhibitor of SIo3 potassium channel and/or the liposomes of the sample fluid and/or the catch fluid are contained in a buffered solution (in other words this means that the sample fluid and/or the catch fluid is(are) buffered solution(s)); and/or
- The at least one inhibitor of SIo3 potassium channel of the sample fluid and/or the catch fluid is present at a concentration of 0.01 mg/L to 5 mg/L, preferably of 0.02 mg/L to 2 mg/L, more preferably of 0.05 mg/L to 1 mg/L, and even more preferably of 0.1 mg/L to 0.2 mg/L, of said fluid.

The sample fluid and/or the catch fluid may be prepared by mixing of the different ingredients.

According to a preferred embodiment, the method according to the invention comprises a further step wherein the catch fluid is replaced by a freezing medium containing liposomes and/or at least one inhibitor of SIo3 potassium channel after sorting. In this case, after collection in the catch fluid and centrifugation, the sperm is diluted in a medium, which is preferably a freezing medium. The freezing medium usually comprises a buffer optionally comprising at least one inhibitor of SIo3 potassium channel, an energy source, at least one freeze protecting agent like glycerol or DMSO, and optionally at least one antibiotic substance, preferably selected but not limited from the group consisting of penicillin, gentamycin, streptomycin, and lincomycin. Any other antibiotic substance determined by the European Union regulations in view of bacterial resistance or other reasons can be chosen. Generally, the freezing medium contains a buffer, several different optional ions, and egg yolk or any other protein source.

According to the invention, a buffer is preferably at least one component selected from the group consisting of Good's buffers, such as TRIS, TES or HEPES and MOPS, and TRIS Good's buffers (also Good buffers) are well known to the skilled person and consist of twenty buffering agents for biochemical and biological research selected and described by Norman Good and colleagues during 1966-1980. TES is 2-[[1,3-dihydroxy-2-(hydroxymethyl)-propan-2-y]amino] ethane sulfonic acid (CAS No. 7365-44-8). HEPES is 2-[4-(2-hydroxyethyl) piperazin-1-yl] ethane sulfonic acid (CAS No. 7365-45-9). MOPS is 3-morpholinopropane-1-sulfonic acid (CAS No. 1132-61-2). TRIS or THAM is 2-amino-2-hydroxymethyl-propan-1,3-diol (CAS No. 77-86-1).

The antioxidant is preferably glutathione. Glutathione is the tripeptide 5-L-glutamyl-L-cysteinylglycine (CAS No. 70-18-8).

As it is known to the skilled person, the sheath fluid is the medium which orients hydrodynamically the sperm cells. The sheath fluid is usually a buffer as defined above. According to a preferred embodiment of the invention, the sheath fluid also contains liposomes and/or at least one inhibitor of SIo3 potassium channel. Said liposomes and/or the at least one inhibitor of SIo3 potassium channel have the characteristics of the liposomes and/or the at least one inhibitor of SIo3 potassium channel present in the sample fluid and/or the catch fluid, that is to say:
- The liposomes of the sheath fluid are contained in a buffered solution (in other words this means that the sheath fluid is a buffered solution); and/or
- The liposomes of the sheath fluid are present at a concentration of 10 g/L to 60 g/L, preferably at a concentration of 30 g/L to 40 g/L, of said fluid; and/or
- The liposomes of the sheath fluid were formed by phosphatidylcholine in a proportion of 20 % to 100 % by weight, preferably in a proportion of 60 % to 80 % by weight, of said fluid; and/or
- More than 95 % of the liposomes of the sheath fluid have a diameter of less than 0.2 µm; and/or
- The at least one inhibitor of SIo3 potassium channel and/or the liposomes of the sheath fluid are contained in a buffered solution (in other words this means that the sheath fluid is a buffered solution); and/or
- The at least one inhibitor of SIo3 potassium channel of the sheath fluid is present at a concentration of 0.01 mg/L to 5 mg/L, preferably of 0.02 mg/L to 2 mg/L, more preferably of 0.05 mg/L to 1 mg/L, and even more preferably of 0.1 mg/L to 0.2 mg/L, of said fluid.

The techniques of the present invention will be readily understood by considering the accompanying drawings:
Figure 1 is a schematic illustration of a flow cytometry sperm sorting device (1) used by the method according to the invention.
Figure 2 is a schematic diagram illustrating the percentage of motility i.e. of motile spermatozoa obtained for each of the solutions tested (the 100% level corresponding to the total population of spermatozoa).
Figure 3 is a schematic diagram illustrating the percentage of viability i.e. of alive spermatozoa obtained for each of the solutions tested (the 100% level corresponding to the total population of spermatozoa).
Figure 4 is a schematic diagram illustrating the percentage of morphology i.e. of normal spermatozoa obtained for each of the solutions tested (the 100% level corresponding to the total population of spermatozoa).

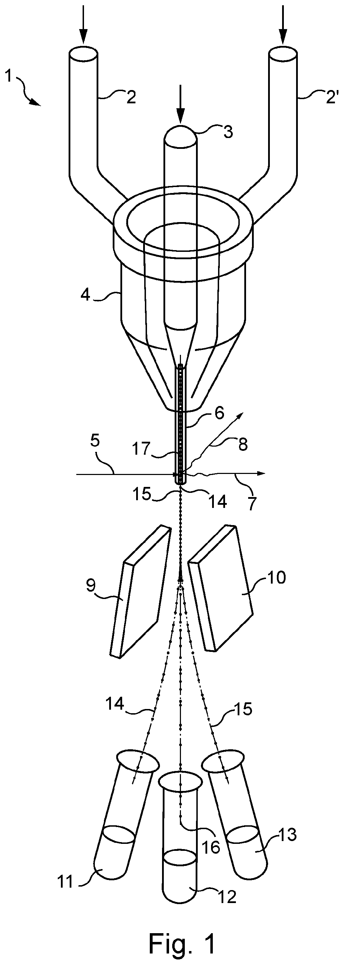

Figure 1 is a schematic illustration of a flow cytometry sperm sorting device (1) used by the method according to the invention. Said sorting device (1) is an in-air droplet system or a flow cell tip (or in-capillary close) system. The unique difference between these systems is that the presence of a capillary duct (17) in the second system, which allows higher speed droplet sorting as it is known to the skilled person.

The device (1) comprises a flow cell (4), two electrodes (9) and (10), and receptacles (11, 12, 13), as well as the electronic and optic and computer devices known to the skilled person.

In particular, the device (1) comprises two ducts (2, 2') for the feed of the sheath fluid, a duct (3) for the feed of the sample fluid, both entering into the flow cell (4) from above. The bottom of the cell flow (4) opens on a duct (6), which can optionally comprise a co-axial capillary tube (17), in which the mixture of the sheath fluid and the sample fluid, comprising the sperm particles to be sorted, enters. The particles to be sorted are capable of travelling upwards of the duct (6) (or the capillary (17)) to receive energy from the laser beam (from a laser source which is not shown) to which they may react according to their DNA as explained above. The laser beam (5) splits into two mutually perpendicular beams (7, 8) which are sensed and processed by optical and electronics (not illustrated). Below the duct (6) (or the capillary (17)) are two charges plates forming electrodes, which will allow the particles to be sorted according to their electrical load which has been transmitted by the laser to them.

Charged particles (+) (14) are driven to fall into the receptacle (11) containing the catch fluid. Charged particles (-) (15) are driven to fall into the receptacle (13) containing the catch fluid. The debris (16) are carried out to fall into the waste receptacle (12).

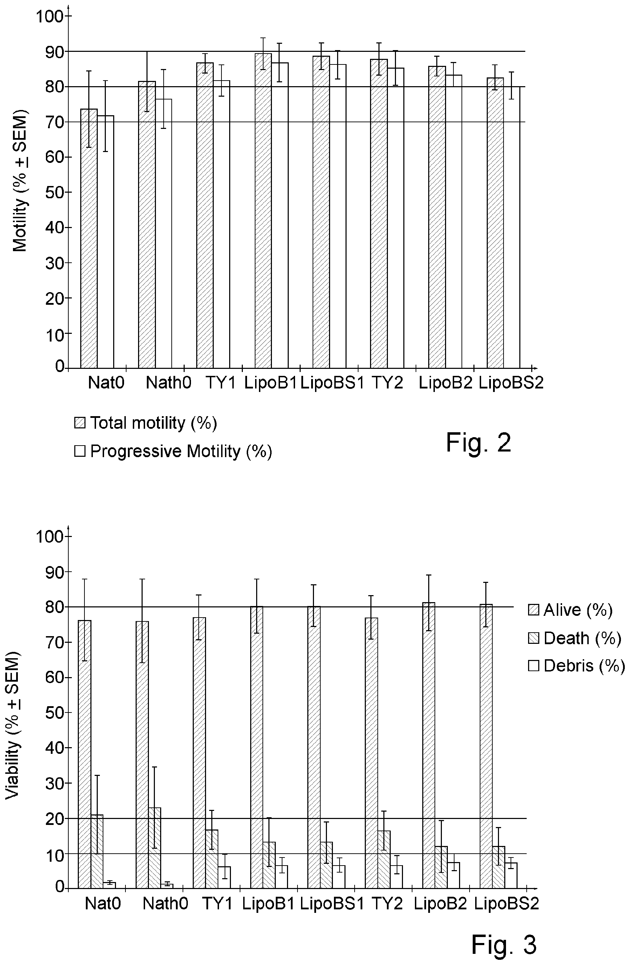

Figures 2, 3 and 4 are explained in the following example.

The invention is further illustrated by the following non-limiting example, which is given for illustrative purposes only.

### EXAMPLE: Liposomes-containing catch fluid for bull semen sorting

### - Material and methods

Ejaculates (n= 10) collected from three Holstein Friesian bulls of proven fertility were used for the experiments. After gross evaluation (volume, colour, consistence and smell), morphology analysis was performed after semen dilution and fixation in Hankcock solution (Formalin 35% (6.25 mL), NaCl solution (150 mL), buffer solution (150 mL) and distilled water (ad 500 mL)).

### Morphology analysis

Approximately 200 sperm cells of each sample were morphologically evaluated under a phase contrast microscope at 1000x magnification.

### Motility (CASA for Computer Assisted Sperm-Analysis) analysis

For this analysis, samples were diluted at 100 x 10⁶/mL. In order to get the right dilution for the evaluation in a CASA system (IVOS apparatus, Hamilton Thorne Bioscience, USA), a total of 10 µL of the diluted sample (pre-warmed for 10 min on a heating stage at 37°C) were mixed with 90µL of Tris-Tes-Buffer (which is the TesT composition disclosed in Graham et al, Journal of dairy Science, Vol 55, No 3, 1972, p. 373, Table 1).

For the CASA measurement, 10 µL of the sample were placed on a Makler counting chamber, which is mounted on a heating stage (37°C) of the Ivos. A total of 10 automatically selected fields were analyzed. After each measurement, the Makler chamber was cleaned with ethanol and a dry tissue.

For the native (*"**Nat**"*) sample, the setup *"Bulle native"* was used. For Hoechst 33342 stained sample *("**Nath**")* sample, the setup *"Bulle Hoechst"* was used. The settings are shown in the following Table.

| | **Bulle Nativ** | **Bulle Hoechst** |
|---|---|---|
| Temperature: | 37°C | 37°C |
| **Image Capture** | | |
| Frames per Sec.: | 60Hz | 60 Hz |
| No of Frames: | 30 | 30 |

| **Cell Detection** | | |
|---|---|---|
| Min. Contrast | 80 | 65 |
| Min. Cell size | 5-8 Pix | 5 Pix |

| **Defaults** | | |
|---|---|---|
| Cell size | 5 Pix | 5 Pix |
| Cell Intensity | 70 | 120 |

| **Progressiv Cells** | | |
|---|---|---|
| Path velocity (VAP): | 15 µm/s | 15 µm/s |
| Straightness (STR): | 30% | 30% |

| **Slow Cells** | | |
|---|---|---|
| Slow Cells | Static | Static |
| VAP Cutoff | 5 µm/s | 5 µm/s |
| VSL Cutoff | 4,5 µm/s | 4,5 µm/s |

| **Illumination** | | |
|---|---|---|
| Intensity | ca. 2355 | ca. 3410 |
| Photometer | ca. 60 | ca. 2 |
| Magnifaction | 10,99 | 10,99 |

| **Video Source** | | |
|---|---|---|
| Hz: | 60 | 60 |
| Field: | Dark | Dark |
| **Static Field Integration Time** | 1 | 1 |
| **Field Selection** | manual | Auto |

### Viability (FACS for Fluorescence-Activated Cell Sorting) analysis

Viability of the sperm samples was tested by flow cytometry (FACS; Gallios analyzer, Beckmann Coulter) analyzing the fluorescence pattern of 20000 sperm cells from each sample.

Samples were diluted with TRIS extender to 50 x 10⁶ sperm/mL and stained with a Life/Dead^{®} sperm viability kit (Fisher Scientific Kit, L7011) containing SYBR^{®} 14 dye (from the company Molecular Probes, Eugene, Oregon, USA) and propidium iodide (PI) (see the paper of Johnson et al. Biology of Reproduction, 53, 276-284, 1995). For each measurement 5 µL of the SYBR^{®} 14 and 3 µL of the Pi, were added to 480 µL TRIS extender and 10 µL of each sperm sample. Tubes were incubated in a Melag incubator at 37°C for 15 min and then analyzed in the flow cytometer.

### - Bull semen sorting using two different liposomes-containing catch fluids according to the invention and one comparative catch fluid

For flow cytometric sperm sorting, samples were diluted to 100.10⁶ sperm /mL, mixed with 25 µL of Hoechst 33342 and incubated at 34 °C for 1h.

### Catch fluids

A liposome-containing solution *("Lipomix")* was prepared. The liposomes were produced from a phospholipid extract, in a buffered saline solution, by extrusion using a mini-extruder from the company Avanti Polar Lipids. The buffered saline solution comprises TRIS (2-amino-2-(hydroxymethyl) propane-1,3-diol (173 mMol), citric acid monohydrate (70.0 mMol) and fructose (56.0 mMol). They were composed of 73% phosphatidylcholine, 11% phosphadiethanolamine and various compounds in lesser quantities (neutral lipids, triglycerides and sphingomyelin). The extracts were hydrated in the buffered saline solution for one night at lipid concentration of 40 g/L. The solution was then treated with ultrasound for 15 minutes (with Elmasonic S 30 H apparatus form the company Elma, at a power of 275 W). At the end, the dispersions were extruded (20 passages per membrane of 100 nm) and the solution thus obtained was the liposome-containing solution (*"Lipomix"*).

Three different catch fluids (0.5 mL/catching tube) were prepared as follow:
- Comparative catch fluid: Catch fluid TRIS-TES buffer (the same as previously) + 2% egg yolk prepared from fresh eggs + 5 µL of seminal plasma (prepared from an ejaculate of a healthy bull by centrifugation and filtration before freezing. (*"**TY**"* sample)).
- First catch fluid according to the invention: Catch fluid TRIS-TES buffer (the same as previously) + 2% Lipomix (*"**LipoB**"* sample).
- Second catch fluid according to the invention: Catch fluid TRIS-TES buffer (the same as previously) + 2% Lipomix + 5 µL of seminal plasma (*"**LipoBS**"* sample).

Immediately after sperm sorting (T1) and 3 h later (T2), CASA, FACS and morphology analyses were performed on the sample corresponding to the X-droplets. For each CASA and FACS analysis, measurements were double checked. For FACS analysis, the setup *"Bulle Hoechst"* was used. For morphology analysis, at least 100 sperm cells were examined under a phase contrast microscope (Olympus BX 60 at x 1000 magnification, supplemented with a heating stage at 37°C).

### Results:

Results of motility (CASA), viability (FACS) and morphology analyses are summarized in Figures 2-4.

Figure 2 is a schematic diagram illustrating the percentage of motility i.e. of motile spermatozoa obtained for each of the solutions tested (the 100% level corresponding to the total population of spermatozoa). The total motility if the percentage of all motile spermatozoa and the progressive motility is the percentage of spermatozoa fulfilling the velocity requirement provided in the Table.

Figure 3 is a schematic diagram illustrating the percentage of viability i.e. of alive spermatozoa obtained for each of the solutions tested (the 100% level corresponding to the total population of spermatozoa). The spermatozoa which are not alive are counted as death spermatozoa (*"death"*) or as partial spermatozoa (*"debris"*)*.*

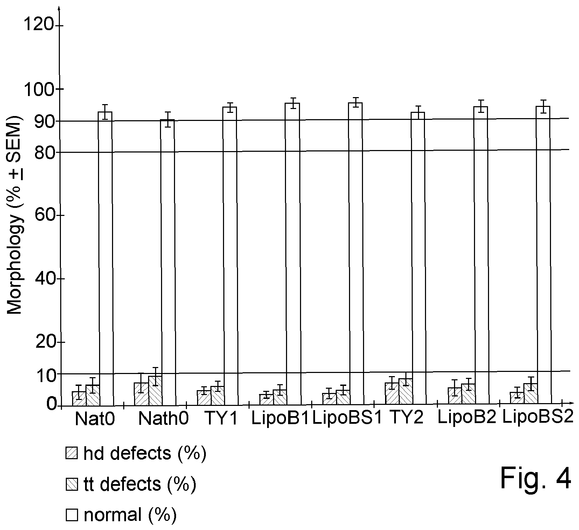

Figure 4 is a schematic diagram illustrating the percentage of morphology i.e. of normal spermatozoa obtained for each of the solutions tested (the 100% level corresponding to the total population of spermatozoa). The spermatozoa which are not normal are counted as spermatozoa having head defects (*"hd defects"*) or as spermatozoa having head defects and other defects (*"tt defects"*)*.*

In conclusion, no difference in semen quality (in terms of morphology, motility and viability) were observed when sex sorted sperm samples were collected in a comparative egg-yolk-containing catch fluid or in a liposome-containing catch fluid according to the invention.

## Claims

1. Method of sperm sorting of the spermatozoa contained in the sperm of a mammalian species, said method comprising a step of mixing the sperm of a mammalian species with liposomes and/or at least one inhibitor of SIo3 potassium channel.

2. Method according to claim 1, wherein said method comprises the following steps:
- mixing the sperm of a mammalian species and a fluorescent dye for labelling sperm DNA with a sample fluid into a fluid mixture;
- providing a flow cytometric sperm sorting device selected from the in-air droplet systems and the flow cell tip close systems;
- using said flow cytometric sperm sorting device on said labelled fluid mixture employing a high energy emitting light source according to the specific excitation spectrum of the dye which is hydrodynamically oriented by the use of sheath fluid, assigning an electric charge corresponding to the chromosome status of each sperm cell and sorting said sperm cell by electrostatic deflection into one of two separate collected tubes each containing a catch fluid;
wherein at least one of the sample fluid and catch fluid comprises liposomes and/or at least one inhibitor of SIo3 potassium channel.

3. Method according to claim 1 or 2, wherein said flow cytometric sperm sorting device is a jet-in-air droplet system.

4. Method according to claim 1 or 2, wherein said flow cytometric sperm sorting device is a flow cell tip close system.

5. Method according to any one of claims 1 to 4, wherein said at least one of the sample fluid and the catch fluid is the sample fluid.

6. Method according to any one of claims 1 to 4, wherein said at least one of the sample fluid and the catch fluid is the catch fluid.

7. Method according to any one of claims 1 to 6, wherein said at least one of the sample fluid and the catch fluid further comprises a buffer, an energy source and optionally at least one antibiotic substance.

8. Method according to claim 7, wherein said at least one of the sample fluid and the catch fluid further contains at least one antioxidant, preferably selected from the group consisting of glutathione, taurine, hypotaurine, pyruvate and cysteine, more preferably glutathione.

9. Method according to claim 8, wherein said antioxidant is preferably present at a concentration ranging from 1 µM/L to 10 µM/L.

10. Method according to any one of claims 1 to 9, wherein the liposomes and/or the at least one inhibitor of SIo3 potassium channel of the sample fluid and/or the catch fluid are contained in a buffered solution.

11. Method according to any one of claims 1 to 10, wherein the liposomes of the sample fluid and/or the catch fluid are present at a concentration of 10 g/L to 60 g/L, preferably at a concentration of 30 g/L to 40 g/L, of said fluid and/or the at least one inhibitor of SIo3 potassium channel of the sample fluid and/or the catch fluid is present at a concentration of 0.01 mg/L to 5 mg/L, preferably of 0.02 mg/L to 2 mg/L, more preferably of 0.05 mg/L to 1 mg/L, and even more preferably of 0.1 mg/L to 0.2 mg/L,of said fluid.

12. Method according to any one of claims 1 to 11, wherein the liposomes of the sample fluid and/or the catch fluid were formed by phosphatidylcholine in a proportion of 20 % to 100 % by weight, preferably in a proportion of 60 % to 80 % by weight, of said fluid.

13. Method according to any one of claims 1 to 12, wherein more than 95 % of the liposomes of the sample fluid and/or the catch fluid have a diameter of less than 0.2 µm.

14. Method according to any one of the claims 1 to 13, comprising a further step wherein the catch fluid is replaced by a freezing medium containing liposomes and/or at least one inhibitor of SIo3 potassium channel after sorting.

15. Method according to claim 14, wherein said freezing fluid comprises a buffer, an energy source, at least one freeze protecting agent, and optionally at least one antibiotic substance, said buffer optionally comprising at least one inhibitor of SIo3 potassium channel.

16. Method according to any one of claims 1 to 15, wherein the sheath fluid contains liposomes and/or at least one inhibitor of SIo3 potassium channel.
